# FASCICULE DE BREVET EUROPEEN

(11) **EP 0 511 187 B1**
(45) Date de publication et mention de la délivrance du brevet: **20.11.1996**
(21) Numéro de dépôt: 92870051.7
(22) Date de dépôt: 30.03.1992
(51) Int. Cl.: C07D 235/08, C07D 235/18, C07D 409/04, C07D 409/14, C07D 405/04, C07D 405/14, C07D 401/04, C07D 401/14

(54) **Procédé de préparation de 1H-benzimidazoles**
Verfahren zur Herstellung von 1H-Benzimidazolen
Process for the preparation of 1H-benzimidazoles

(30) Priorité: 18.04.1991 GB 9108279
(43) Date de publication de la demande: 28.10.1992
(73) Titulaire: U C B, S.A., B-1050 Bruxelles (BE)
(72) Inventeur: van Gysel, August, B-1710 Dilbeek (BE); Maquestiau, André, B-7000 Mons (BE); Vanden Eynde, Jean-Jacques, B-7000 Mons (BE); Mayence, Annie, B-7000 Mons (BE); Vanovervelt, Jean-Claude, B-7548 Warchin (BE)
(74) Mandataire: Dusseldorp, Raymond

(56) Documents cités:
- SYNTHESIS. vol. 1988, no. 3, 1 Mars 1988, STUTTGART DE pages 244 - 246; ALBA CHIMIRRI,A.O.: 'A Simple, One-Pot Synthesis of Novel 1H,3H-Thiazolo-[3,4- a]benzimidazoles'
- SOVIET INVENTIONS ILLUSTRATED Section Ch, Week 8649, 18 Décembre 1986 Derwent Publications Ltd., London, GB; Class C, AN 86-325044/49 & SU-A-1227629
- BERICHTE DER DEUTSCHEN CHEMISCHEN GESELLSCHAFT. vol. 75, no. 12, 10 Février 1942, WEINHEIM DE pages 1936 - 1948; RUDOLF WEIDENHAGEN, U.A.: 'über eine neue Darstellung von N-alkylierten Imidazoloverbindungen'
- BERICHTE DER DEUTSCHEN CHEMISCHEN GESELLSCHAFT. vol. 69B, no. 10, 7 Octobre 1936, WEINHEIM DE pages 2263 - 2272; RUDOLF WEIDENHAGEN: 'Eine neue Synthese von Benzimidazol-Derivaten'
- JOURNAL OF HETEROCYCLIC CHEMISTRY. vol. 2, no. 4, Décembre 1965, PROVO US pages 453 - 456; H. F. RIDLEY, A.O.: 'A New Synthesis of Benzimidazoles and Aza- analogs'
- BULLETIN DES SOCIETES CHIMIQUES BELGES. vol. 96, no. 9, Septembre 1987, OXFORD GB pages 719 - 720; ERNST ANDERS, A.O.: 'N-(1-Halogenoalkyl)Heteroaryliumsalze Synthese und Reaktionen'
- BULLETIN DES SOCIETES CHIMIQUES BELGES. vol. 98, no. 8, Août 1989, OXFORD GB pages 523 - 528; A. MAQUESTIAU, A.O.: 'Synthesis of Imines from N-(1- Halogenoalkyl)Heteroarylium Salts'
- JOURNAL OF ORGANIC CHEMISTRY. vol. 54, no. 20, 29 Septembre 1989, EASTON US pages 4808 - 4812; ERNST ANDERS, A.O.: 'N-(1-Haloalkyl)pyridinium alts: Preparation and Use for New Syntheses of Other N-(1-Substituted- alkyl)pyridinium Salts, N,N'-(1-Alkylidene)bisamines, and N,N'-(1- Alkylidene)bisbenzazoles'

## Description

La présente invention se rapporte à un procédé nouveau et avantageux pour la préparation de 1H-benzimidazoles substitués en position 2, à partir d'une 1,2-benzènediamine et d'un aldéhyde.

Les 1H-benzimidazoles sont des produits chimiques qui ont de nombreuses applications commerciales, notamment comme agents anthelminthiques et fongicides, comme produits de départ pour la fabrication de produits pharmaceutiques et cosmétiques, comme produits utiles dans les procédés photographiques en couleur, comme catalyseurs dans la fabrication de polyuréthannes, comme produits de départ de polymères organiques à haute résistance thermique et de fibres à haute performance, etc.

Ainsi par exemple, le 2-phényl-1H-benzimidazole est un agent anthelminthique (brevet américain 3.549.754) et fongicide (demande de brevet japonais 29.934/78). Il peut également servir comme matière première pour la préparation de l'acide 2-phényl-1H-benzimidazole-5-sulfonique (V.G. SAYAPIN et al., Khim. Géterotsikl. Soedin, 1970,681-683; Chem. Abstr. 73, (1970), réf. 45409p) qui est largement utilisé comme filtre des rayons ultraviolets dans de nombreuses compositions solaires anti-UV (brevets américains 4.671.955 et 4.707.354).

Le 2-phényl-1H-benzimidazole, le 2-n-pentyl-1H-benzimidazole, le 2-(2-chlorophényl)-1H-benzimidazole, le 2-(4-méthylphényl)-1H-benzimidazole et le 2-(2-thiényl)-1H-benzimidazole sont utiles dans des procédés photographiques en couleur (brevet belge 844.179). Ce dernier composé, de même que le 2-(3-thiényl)-1H-benzimidazole, le 2-(2-chlorophényl)-1H-benzimidazole, le 2-(2-furanyl)-1H-benzimidazole, le 2-(3-furanyl)-1H-benzimidazole, le 2-(2-pyridinyl)-1H-benzimidazole et le 2-(3-pyridinyl)-1H-benzimidazole possèdent en outre des propriétés anthelminthiques (brevet américain 3.549.754).

Le 2-(4-nitrophényl)-1H-benzimidazole (demande de brevet japonais 29.934/78) et le 2-(2-furanyl)-1H-benzimidazole (fuberidazole) sont des fongicides, tandis que le 5-chloro-2-(4-fluorophényl)-1H-benzimidazole est un agent antiparasitaire, antibactérien et antifongique (M. RIDI et al., Boll.Chim.Farm., 107,(1968), 667-674).

Les 2-alkyl-1H-benzimidazoles peuvent être utilisés en tant que catalyseurs dans la fabrication de polyuréthannes (brevet britannique 1.491.219) ou comme produits intermédiaires pour la préparation de produits pharmaceutiques ou cosmétiques (brevet suisse 653.022 et demande de brevet japonais 125.537/74).

Le 2-(phénylméthyl)-1H-benzimidazole (bendazol) est un médicament qui possède des propriétés spasmolytiques et vasodilatatrices (The Merck Index, 11e édition, 1989, page 161, sous le n° 1043), tandis que le 5-chloro-2-(4-chlorophényl)-1H-benzimidazole possède des propriétés analgésiques (G. PAGLIETTI et al., Il Farmaco, Ed.Sci., 43,(1988),215-226). Le 2,2′-(1,3-phénylène)bis(1H-benzimidazole) et le 2,2′-(1,4-phénylène)bis(1H-benzimidazole) servent à la préparation de polycétones résistant à la chaleur (demandes de brevets japonais 134.797/74 et 134.796/74).

L'acide 2-(4-carboxyphényl)-1H-benzimidazole-5-carboxylique est employé comme monomère dans la fabrication de fibres à haute résistance (brevet russe 503.864). D'autre part, l'acide 2,2′-(1,4-phénylène)bis(1H-benzimidazole-5-carboxylique) (demande de brevet japonais 15826/72), l'acide 2,2′-(1,3-phénylène)bis(1H-benzimidazole-5-carboxylique), de même que l'acide 4,4′-[5,5′-bi-1H-benzimidazole]-2,2′-diylbis(benzoïque) (R.A. BRAND et al., J.Polym.Sci.,Polym.Chem.Ed.17,(1979),1145-1152) sont des monomères pour la synthèse de polymères thermostables.

Il convient aussi de mentionner le 5-nitro-2-(4-nitrophényl)-1H-benzimidazole (V.STERBA et al., Collect.Czech.Chem.Commun.,31, (1965),1093) qui par réduction fournit la 2-(4-aminophényl)-1H-benzimidazol-5-amine, laquelle sert de matière première dans la fabrication des polyimides aromatiques (polybenzimidazoles) qui sont considérées comme l'une des classes de polymères organiques présentant la meilleure résistance à la chaleur et à l'oxydation (L.W. FROST et al.,J.Polym.Sc.,6,(1968), 215-233). Par conséquent, ces composés trouvent un débouché dans la fabrication de fibres à haute performance, notamment pour la substitution de l'amiante dans les applications où des propriétés ignifuges sont de première importance (HOECHST A.G. Flame Retardant Fibers at 3rd TECHTEXTIL, JTN September (1989), 72-74).

Quant aux 5-chloro-2-(4-cyanophényl)-1H-benzimidazole, 5-chloro-2-(4-nitrophényl)-1H-benzimidazole et 5-nitro-2-(4-chlorophényl)-1H-benzimidazole, ce sont aussi des matières premières intéressantes pour la préparation de polybenzimidazoles à haute stabilité thermique. Par réduction des fonctions cyano et nitro de ces molécules, selon des méthodes connues en soi, on obtient les composés aminés correspondants. Ces amines chlorées sont des monomères de départ pour la préparation de polybenzimidazoles soit par homopolymérisation entre des molécules de même nature, soit par copolymérisation avec des monomères classiques utilisés aussi dans l'industrie des polymères thermostables, tels que par exemple la 4,4′-diaminodiphénylsulfone, la m-xylylènediamine ou le bisphénol A, ou encore la 4,4′-dihydroxydiphénylsulfone; ce dernier composé conduit à des polyéthersulfones dans lesquels des structures benzimidazoles sont incluses. De même, le 2,2′-(1,4-phénylène)bis(5-chloro-1H-benzimidazole) et le 2,2′-bis(4-chlorophényl)-5,5′-bi-1H-benzimidazole sont des monomères utiles pour la préparation de polybenzimidazoles par copolymérisation avec les monomères diaminés ou dihydroxylés cités ci-dessus, par exemple la 4,4′-dihydroxydiphénylsulfone, seuls ou en mélange avec la 4,4'-dichlorodiphénylsulfone.

Il existe déjà différents procédés de synthèse des 1H-benzimidazoles substitués en position 2; ceux-ci sont notamment décrits dans l'ouvrage "THE CHEMISTRY OF HETEROCYCLIC COMPOUNDS "(A. WEISSBERGER et E.C. TAYLOR), 1981, volume 40, "Benzimidazoles and Congeneric tricyclic compounds", Partie I (P.N. PRESTON), pages 6 à 60.

Parmi ces procédés, les plus intéressants sont ceux qui partent d'une 1,2-benzènediamine d'une part, et d'un acide carboxylique ou d'un aldéhyde, d'autre part.

Toutefois, les méthodes de synthèse disponibles jusqu'à présent ne permettent pas de préparer les 1H-benzimidazoles de manière aisée et économique.

Une de celles-ci, proposée par A. LADENBURG (Chem.Ber.,8,(1875),677) et développée plus tard par M.A. PHILLIPS (J.Chem.Soc.,1928,2393 et 3134; J.Chem.Soc.,1929,2820 et J.Chem.Soc.1931,1143) consiste à faire réagir à température élevée une 1,2-benzènediamine avec un acide carboxylique ou son anhydride, en présence d'acide chlorhydrique aqueux.

Toutefois, cette méthode est d'application limitée parce que les acides aromatiques et les acides aliphatiques ramifiés ne réagissent pas ou réagissent difficilement et les rendements obtenus sont souvent très faibles. Pour parer à cet inconvénient, des conditions expérimentales plus drastiques sont nécessaires, par exemple chauffage en tube scellé à 180-190°C, sous pression (B.A. PORAI-KOSHITS et al., Zhur.Obshchei Khim,17,(1947), 1768-1773 et 19,(1949),1545-1552(Chem.Abstr.42,(1948), 5903c et 44,(1950),1100b). Pour citer un exemple, cette méthode a permis de préparer le 2-(3-pyridinyl)-1H-benzimidazole avec un rendement de 40% en tube scellé à 185°C dans l'acide chlorhydrique dilué (B.A. PORAI-KOSHITS et al. (1947), loc.cit.), et aussi, en présence d'acide polyphosphorique à 250°C, mais avec un rendement qui n'atteint que 18% (D.W. HEIN et al.,J.Am.Chem.Soc.,79,(1957),427).

Des sélénamides ont également été proposés pour remplacer les acides carboxyliques dans cette réaction (V.I. COHEN, J.Heterocycl. Chem.,16,(1979),13). Le rendement signalé atteint 50%, mais les sélénamides de départ sont coûteux, toxiques et peu courants.

Une méthode plus intéressante consiste à effectuer la synthèse des 1H-benzimidazoles substitués en position 2 à partir d'une 1,2-benzènediamine et d'un aldéhyde. Toutefois cette méthode présente aussi de nombreux inconvénients, notamment les rendements sont faibles et la réaction conduit fréquemment à la formation de mélanges inséparables de benzimidazoles 2-mono- et 1,2-disubstitués. En outre, la réaction fournit souvent une imine en équilibre avec sa forme cyclisée benzimidazoline qu'il faut transformer en un benzimidazole par oxydation dans un stade ultérieur. Cette oxydation est généralement réalisée en présence d'un agent oxydant, tel que l'acétate de cuivre (R.WEIDENHAGEN et al., Chem.Ber.,75,(1942),1936-1948), le tétraacétate de plomb (F.F. STEPHENS et al., J.Chem.Soc.1949,2971), le manganate de baryum (R.G. SRIVASTAVA et al.,Syn.Comm.,18,(1988),1537), l'anhydride sulfureux gazeux (brevet russe 1.227.629) ou encore le dioxyde de manganèse (R.G. SRIVASTAVA et al., loc.cit.). Dans des cas rares et souvent particuliers, l'oxydation de l'imine en benzimidazole, via la benzimidazoline, a pu être obtenue par chauffage (F.F. STEPHENS et al.,J.Chem.Soc.,1950,1722) ou par exposition à l'air (J.G. SMITH et al.,Tetrahedron Lett.,38,(1971),3541).

Quoi qu'il en soit, dans la majorité des cas, plusieurs manipulations sont nécessaires pour isoler le composé benzimidazolique.

A titre d'exemple, c'est par cette seconde méthode qu'a été préparé le 2-(2-furanyl)-1H-benzimidazole avec un rendement de 65% (R. WEIDENHAGEN, Chem.Ber.,69B,(1936),2263-2272). Toutefois la récupération du produit souhaité à partir du sel de cuivre du 1H-benzimidazole obtenu selon cette méthode est très difficile.

Une variante de cette dernière méthode a été proposée par H.F.RIDLEY et al., (J.Heterocycl.Chem.,2,(1965),453-456). Elle consiste à faire réagir la 1,2-benzènediamine non pas directement avec l'aldéhyde, mais avec le produit d'addition bisulfitique de cet aldéhyde. Bien que présentant divers avantages par rapport au procédé initial, notamment une température de réaction moins élevée, cette variante nécessite néanmoins au préalable la préparation et l'isolement du produit d'addition bisulfitique de l'aldéhyde. Cette variante comporte donc nécessairement au moins deux stades.

De ce qui précède il ressort que :
a) il n'y a pas jusqu'à présent de méthode générale permettant de préparer les 1H-benzimidazoles substitués en position 2 directement à partir d'une 1,2-benzènediamine et d'un aldéhyde en un seul stade ;
b) les rendements obtenus sont en général faibles et dépassent rarement 50%;
c) les conditions de température et de pression sont souvent élevées;
d) les 1H-benzimidazoles obtenus sont difficilement séparables du milieu de réaction.

Par conséquent, il est donc intéressant de découvrir un procédé de préparation de 1H-benzimidazoles substitués en position 2 plus simple, permettant de préparer ces composés au départ des mêmes matières premières, mais en un seul stade et avec de meilleurs rendements et sans les inconvénients des procédés actuellement connus.

Il a été trouvé maintenant que les 1H-benzimidazoles substitués en position 2 peuvent être préparés à partir d'une 1,2-benzènediamine et d'un aldéhyde de manière aisée et beaucoup plus avantageuse quant aux rendements obtenus en faisant réagir, en présence d'un agent fixateur d'acide, le produit formé par la réaction entre un aldéhyde, un halogénure de thionyle et un composé hétérocyclique aromatique azoté choisi dans le groupe consistant en la pyridine, la pyrimidine, la quinoléine, l'isoquinoléine, la pyrazine et le 1-méthylimidazole avec une 1,2-benzènediamine éventuellement substituée.

La présente invention a donc pour objet un procédé de préparation de 1H-benzimidazoles substitués en position 2 à partir d'une 1,2-benzènediamine et d'un aldéhyde, qui est caractérisé en ce que l'on fait d'abord réagir ensemble un aldéhyde avec un halogénure de thionyle et un composé hétérocyclique aromatique azoté choisi dans le groupe consistant en la pyridine, la pyrimidine, la quinoléine, l'isoquinoléine, la pyrazine et le 1-méthylimidazole, en ce que l'on fait réagir ensuite, en présence d'un agent fixateur d'acide le produit ainsi formé avec une 1,2-benzènediamine éventuellement substituée et en ce que l'on sépare le 1H-benzimidazole ainsi obtenu du mélange de réaction.

Le procédé de la présente invention convient pour la préparation d'un grand nombre de 1H-benzimidazoles substitués en position 2 et comportant les substituants les plus divers sur le noyau benzénique. Ainsi par exemple, si l'on utilise un aldéhyde comportant une seule fonction aldéhyde, on obtient un 1H-benzimidazole ne contenant qu'un seul groupe benzimidazole; par contre, si on utilise un aldéhyde comportant deux fonctions aldéhyde, on obtient un composé symétrique qui contient deux groupes benzimidazoles, c'est-à-dire un bis(1H-benzimidazole).

C'est pourquoi la présente invention procure de préférence un procédé de préparation de 1H-benzimidazoles substitués en position 2 répondant à la formule générale 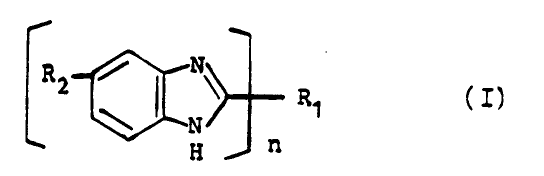 dans laquelle
- R₁: représente un radical alkyle linéaire ou ramifié contenant 1 à 13 atomes de carbone, un radical phényle, halogénophényle, 4-nitrophényle, 4-méthylphényle, 4-cyanophényle, 4-carboxyphényle, benzyle, thiényle, furanyle, pyridinyle ou un radical 1,3- ou 1,4-phénylène,
- R₂: représente un atome d'hydrogène ou de chlore, un groupe nitro ou carboxyle, le groupe 2-(4-chlorophényl)-1H-benzimidazol-5-yle ou le groupe 2-(4-carboxyphényl)-1H-benzimidazol-5-yle, et
- n: est égal à 1 ou 2,
avec les restrictions que :
a) lorsque R₁ représente un radical alkyle, phényle, 4-méthylphényle, benzyle, thiényle, furanyle ou pyridinyle, R₂ est de l'hydrogène et n = 1,
b) lorsque R₁ représente un radical halogénophényle ou 4-nitrophényle, R₂ est un atome d'hydrogène ou de chlore ou un groupe nitro et n = 1,
c) lorsque R₁ représente le radical 4-cyanophényle, R₂ représente un atome de chlore et n = 1,
d) lorsque R₁ représente le radical 4-carboxyphényle, R₂ représente un groupe carboxyle ou le groupe 2-(4-carboxyphényl)-1H-benzimidazol-5-yle, et n = 1,
e) lorsque R₁ est un radical 1,3- ou 1,4-phénylène, R₂ représente un atome d'hydrogène ou de chlore ou un groupe carboxyle et n = 2, et
f) lorsque R₂ est le groupe 2-(4-chlorophényl)-1H-benzimidazol-5-yle, R₁ représente le radical 4-chlorophényle et n = 1,
caractérisé en ce que l'on fait réagir ensemble un aldéhyde de formule générale

R₁(CHO)ₙ (II)

dans laquelle R₁ et n ont la signification donnée ci-dessus, avec un halogénure de thionyle et un composé hétérocyclique aromatique azoté choisi dans le groupe consistant en la pyridine, la pyrimidine, la quinoléine, l'isoquinoléine, la pyrazine et le 1-méthylimidazole, en ce que l'on fait réagir ensuite, en présence d'un agent fixateur d'acide, le produit formé (a) avec une 1,2-benzènediamine de formule 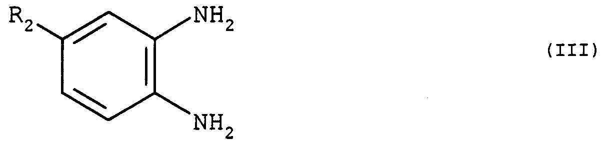 dans laquelle R₂ représente un atome d'hydrogène ou de chlore ou un groupe nitro ou carboxyle lorsqu'on désire obtenir un 1H-benzimidazole de formule I dans laquelle R₂ représente un atome d'hydrogène ou de chlore ou un groupe nitro ou carboxyle, ou (b) avec la [1,1'-biphényl]-3,3',4,4'-tétramine lorsqu'on désire obtenir un 1H-benzimidazole de formule I dans laquelle R₂ représente le groupe 2-(4-chlorophényl)-1H-benzimidazol-5-yle ou le groupe 2-(4-carboxyphényl)-1H-benzimidazol-5-yle et en ce qu'on sépare le 1H-benzimidazole ainsi obtenu du mélange de réaction. La réaction du procédé conforme à l'invention, qui peut être représentée par le schéma suivant : 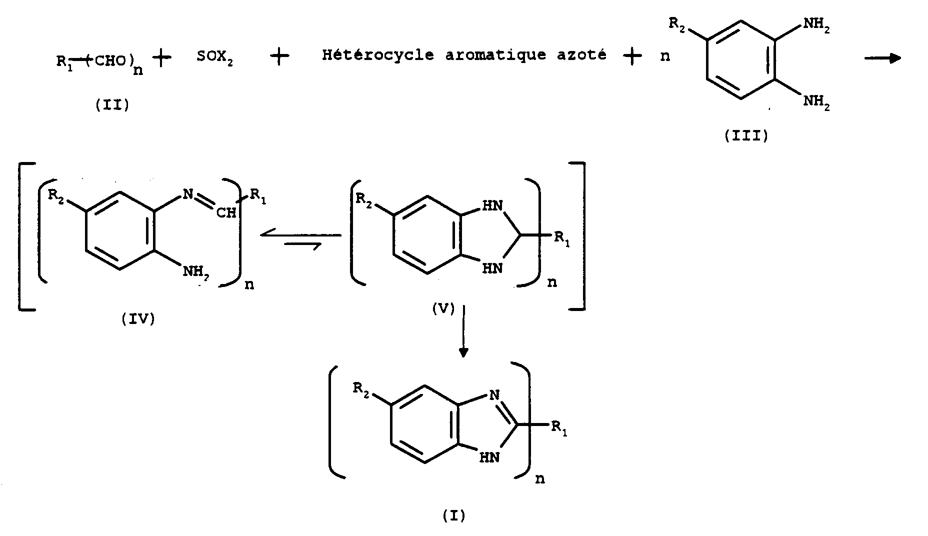 passe par la formation transitoire d'une imine de formule IV (ou de sa forme cyclique benzimidazoline de formule V) qui s'oxyde in situ pour fournir finalement les benzimidazoles de formule I. Dans ces formules, les symboles R₁ et n ont la signification donnée ci-dessus, R₂ représente un atome d'hydrogène ou de chlore ou un groupe nitro ou carboxyle et X représente un atome d'halogène, de préférence un atome de chlore.

Il était tout à fait imprévisible que cette réaction puisse fournir directement le 1H-benzimidazole de formule I. En effet, dans la méthode de synthèse classique mettant en jeu un aldéhyde et une 1,2-benzènediamine, la réaction conduit à l'imine de formule IV, qu'il faut ensuite transformer en un 1H-benzimidazole de formule I au moyen d'un agent oxydant.

Conformément à l'invention, on exécute la réaction éventuellement dans un solvant inerte, à l'exclusion de l'eau et des solvants hydroxylés, à une température comprise entre 0°C et la température d'ébullition du solvant et en présence d'un agent fixateur d'acide qui peut être la 1,2-benzènediamine de formule III utilisée en excès. Dans ce dernier cas, on utilise au moins deux moles de la 1,2-benzènediamine par mole d'aldéhyde mise en oeuvre lorsque cet aldéhyde comporte une seule fonction aldéhyde (n=1 dans la formule II) et de préférence au moins quatre moles de la 1,2-benzènediamine par mole d'aldéhyde lorsque cet aldéhyde comporte deux fonctions aldéhyde (n=2 dans la formule II). En outre, on peut aussi éventuellement opérer en atmosphère de gaz inerte, par exemple de l'azote, avant l'addition de la 1,2-benzènediamine, mais cette précaution n'est pas indispensable.

Le solvant inerte dans lequel on peut effectuer la réaction, peut être un hydrocarbure aliphatique ou cycloaliphatique, comme par exemple l'hexane ou le cyclohexane, un hydrocarbure aliphatique chloré, comme par exemple le dichlorométhane, le tétrachloroéthylène ou le 1,1,1-trichloroéthane, un éther aliphatique comme le diéthyléther et le 1,2-diméthoxyéthane ou un hydrocarbure aromatique chloré ou non, tel que le toluène, le chlorobenzène ou le dichlorobenzène.

En tant qu'halogénure de thionyle on utilisera surtout le chlorure ou le bromure de thionyle, de préférence le chlorure de thionyle.

En tant que composé hétérocyclique aromatique azoté utilisable dans le procédé conforme à l'invention, on préfère la pyridine. Les composés hétérocycliques aromatiques azotés peuvent éventuellement jouer le rôle de solvant pour effectuer la réaction. Dans ce cas, ces composés seront employés en large excès et l'utilisation d'un solvant inerte auxiliaire est superflue.

Les aldéhydes de formule II qui peuvent être utilisés comme produits de départ dans la préparation des 1H-benzimidazoles de formule I sont des aldéhydes aliphatiques contenant 2 à 14 atomes de carbone ou des aldéhydes aromatiques substitués ou non ou hétéroaromatiques. A titre d'exemples on citera l'acétaldéhyde, le propionaldéhyde, le butyraldéhyde, le 2-méthylpropionaldéhyde, le 2,2-diméthylpropionaldéhyde, le n-décanal, le benzaldéhyde, le 2-chlorobenzaldéhyde, le 4-chlorobenzaldéhyde, le 4-nitrobenzaldéhyde, le 4-méthylbenzaldéhyde, le 4-cyanobenzaldéhyde, le phénylacétaldéhyde, l'acide 4-formylbenzoïque, le 1,3- ou 1,4-benzènedicarboxaldéhyde, le 2- ou 3-pyridinecarboxaldéhyde, le 2- ou 3-furanecarboxaldéhyde, le 2- ou 3-thiophènecarboxaldéhyde, etc. .

Les 1,2-benzènediamines de formule III qui peuvent être utilisées comme produits de départ dans le procédé conforme à l'invention sont des 1,2-benzènediamines substituées ou non. A titre d'exemples on citera la 1,2-benzènediamine, la 4-chloro-1,2-benzènediamine, la 4-nitro-1,2-benzènediamine, l'acide 3,4-diaminobenzoïque, etc.. On utilise la [1,1′-biphényl]-3,3′,4,4′-tétramine lorsqu'on désire obtenir un 5,5′-bi-1H-benzimidazole substitué dans les positions 2 et 2′.

Lorsque la réaction est terminée, le produit souhaité précipite généralement dans le milieu réactionnel en même temps que le sel de la 1,2-benzènediamine utilisée en excès. Pour séparer le 1H-benzimidazole obtenu il suffit donc simplement d'isoler le précipité par filtration et de le laver soigneusement avec de l'eau pour le débarrasser des sels solubles. Le composé hétérocyclique aromatique azoté et le solvant organique peuvent être récupérés aisément par exemple par distillation, et recyclés.

La neutralisation de la phase aqueuse permet de récupérer, selon les procédés conventionnels, l'excès de 1,2-benzènediamine qui peut être recyclé dans de nouvelles réactions.

Dans le cas où seul le sel de la 1,2-benzènediamine précipite dans le milieu réactionnel, celui-ci est séparé par filtration, le solvant est évaporé sous pression réduite et le résidu qui renferme le 1H-benzimidazole souhaité est lavé à l'eau.

Selon une autre forme de réalisation de l'invention, on peut remplacer par de l'acétate de sodium l'excédent de 1,2-benzènediamine nécessaire pour fixer l'acide qui se forme pendant la réaction. Dans cette forme de réalisation, on utilise seulement une mole de la 1,2-benzènediamine par mole d'aldéhyde. Ceci simplifie le mode opératoire et supprime du même coup les pertes inévitables liées à la récupération de la 1,2-benzènediamine de départ. Selon ce mode de réalisation, on ajoute donc au milieu réactionnel, par mole d'aldéhyde, seulement une mole de la 1,2-benzènediamine et une solution aqueuse ou alcoolique contenant deux moles d'acétate de sodium pour compléter la réaction avec la 1,2-benzènediamine L'acétate de sodium neutralise au fur et à mesure de sa formation le sel de la 1,2-benzènediamine mise en oeuvre, permettant ainsi à la diamine libérée de réagir à nouveau jusqu'à ce que la réaction soit complète.

Dans ce mode de réalisation, l'ensemble des opérations (mélange des réactifs) peut aussi être réalisé en l'absence de refroidissement externe destiné à maintenir constante la température du milieu réactionnel. En outre, le temps total nécessaire pour effectuer la réaction peut être considérablement raccourci, d'un tiers au moins, si le mélange réactionnel est porté à reflux après l'addition de la solution d'acétate de sodium.

La réaction entre un aldéhyde, un halogénure de thionyle et un composé hétérocyclique aromatique azoté est connue. On a montré récemment qu'il se forme un sel d'ammonium quaternaire; ainsi par exemple, le 4-méthylbenzaldéhyde réagit avec le chlorure de thionyle et la pyridine, à une température voisine de -50°C, en formant le chlorure de N-(4-méthyl-α-chlorobenzyl)pyridinium (E. ANDERS et J.G. TROPSCH, Bull.Soc.Chim.Belg., 96,(1987),719-720). Divers composés hétérocycliques aromatiques azotés ont été utilisés. C'est ainsi que les auteurs précités ont réussi à préparer des sels d'ammonium quaternaires à partir de pyridine, d'isoquinoléine, de 1-méthylimidazole et de dérivés alkylés de la pyridine. Ils ont aussi montré que ces sels peuvent réagir avec divers réactifs nucléophiles tels que le fluorure de césium, la triphénylphosphine, le bisulfite de sodium, le phtalimide potassique et le trithiocarbonate de potassium, par substitution de l'atome d'halogène du radical halogénoalkyle.

En outre, A. MAQUESTIAU et al.,(Bull.Soc.Chim.Belg.,98,(1989),523-528) ont utilisé ces sels pour préparer des imines, en particulier en faisant réagir dans des conditions douces le chlorure de N-(α-chlorobenzyl)pyridinium ou de N-(α-chlorobenzyl)pyrimidinium avec une amine primaire.

Enfin, plus récemment, E. ANDERS et al., (J.Org.Chem.54, (1989),4808-4812) ont montré qu'une amine secondaire et un halogénure de N-(1-halogénoalkyl)pyridinium réagissent avec substitution successive de l'atome d'halogène et du groupe pyridinium et formation d'un composé symétrique. En particulier, ces auteurs ont préparé des N,N′-(1,1-alkylidène)bis(1H-benzimidazoles) lorsque l'amine secondaire est le 1H-benzimidazole.

Toutefois, à la connaissance de la demanderesse, la littérature n'enseigne pas que la réaction précitée entre un aldéhyde, un halogénure de thionyle et un composé hétérocyclique aromatique azoté peut avantageusement être utilisée pour la synthèse de 1H-benzimidazoles, ni qu'elle permet de préparer les 1H-benzimidazoles avec oxydation simultanée de l'imine intermédiaire sans nécessiter une opération supplémentaire de traitement par un agent oxydant.

Les avantages du procédé conforme à l'invention par rapport aux procédés connus de préparation des 1H-benzimidazoles sont multiples :
a) le procédé de l'invention peut se faire en un seul stade: synthèse des 1H-benzimidazoles directement à partir des aldéhydes et des 1,2-benzènediamines appropriés, alors que les procédés connus au départ des mêmes matières premières sont beaucoup plus élaborés et exigent au moins deux stades en passant par une imine intermédiaire qu'il faut transformer en le benzimidazole souhaité par oxydation avec un agent oxydant ;
b) les conditions opératoires du procédé de l'invention sont beaucoup plus douces que celles des procédés de l'état de la technique ;
c) les rendements obtenus sont plus élevés et sont d'au moins 45% et peuvent facilement atteindre de 90 à 95% dans les cas les plus favorables ;
d) on n'utilise pas de réactifs (agents oxydants) ou de solvants coûteux et la séparation des 1H-benzimidazoles obtenus à partir du mélange de réaction se fait avec une très grande facilité; il en est de même en ce qui concerne la récupération du composé hétérocyclique aromatique azoté, du solvant organique et de l'excès de diamine éventuellement utilisé.

Les exemples qui suivent illustrent l'invention sans la limiter.

### Exemple 1. Préparation du 2-phényl-1H-benzimidazole.

a) A 12 ml d'une solution 1 molaire de chlorure de thionyle dans le dichlorométhane, refroidie à la température de 0°C, et sous atmosphère inerte (azote), on ajoute successivement 6 ml d'une solution 2 molaire de pyridine dans le dichlorométhane et 5 ml d'une solution 2 molaire (10 mmoles) de benzaldéhyde dans le dichlorométhane. On agite le mélange à 0°C pendant une heure. Ensuite, on ajoute lentement 3,24 g (30 mmoles) de 1,2-benzènediamine et on maintient l'agitation pendant plusieurs heures tout en laissant le mélange réactionnel revenir à la température ambiante. On filtre le précipité qui s'est formé pendant la réaction et on le lave avec du dichlorométhane. Ensuite, ce précipité est lavé soigneusement avec de l'eau pour dissoudre les sels d'amine qu'il contient. On obtient ainsi le 2-phényl-1H-benzimidazole de P.F. 287-290°C. Rendement: 70%.
b) Le même produit peut aussi être préparé comme indiqué en a) ci-dessus sans opérer sous atmosphère inerte. Le rendement est de 70% et le produit isolé est identique au produit préparé en a).
   Le même produit peut encore être préparé selon l'une des autres formes de réalisation indiquées ci-après.
c) A une solution contenant 8 ml (110 mmoles) de chlorure de thionyle dans 100 ml de dichlorométhane, on ajoute successivement, sous agitation, 9 ml (110 mmoles) de pyridine et 10 ml (100 mmoles) de benzaldéhyde, tout en maintenant la température du milieu réactionnel entre 5 et 10°C. Après l'addition on agite le mélange pendant une heure, puis on ajoute successivement 10,8 g (100 mmoles) de 1,2-benzènediamine et ensuite, goutte à goutte, 50 ml d'une solution aqueuse contenant 16,4 g (200 mmoles) d'acétate de sodium. On maintient l'agitation pendant une nuit tout en laissant le mélange réactionnel revenir lentement à la température ambiante. On filtre le précipité qui s'est formé, on le lave à l'eau et on sèche. On obtient 18 g de 2-phényl-1H-benzimidazole identique au produit préparé en a) ci-dessus.
   Rendement: 93%.
d) A 12 ml d'une solution 1 molaire de chlorure de thionyle dans le dichlorométhane, refroidie à la température de 0°C, on ajoute successivement 6 ml d'une solution 2 molaire de pyrimidine dans le dichlorométhane et 5 ml d'une solution 2 molaire (10 mmoles) de benzaldéhyde dans le dichlorométhane. On agite le mélange à 0°C pendant six heures et on procède ensuite comme en a) ci-dessus.
   Rendement: 65%.
e) On procède comme en a) ci-dessus, mais on utilise une solution 1 molaire de bromure de thionyle.
   Rendement: 60%.
f) On procède comme en a) ci-dessus, mois on utilise une solution 2 molaire de quinoléine au lieu de pyridine.
   Rendement: 50%.
g) On procède comme en a) ci-dessus, mois on utilise une solution 2 molaire d'isoquinoléine.
   Rendement: 55%.
h) On procède comme en a) ci-dessus, mois on utilise une solution 2 molaire de 1-méthylimidazole.
   Rendement: 60%.
i) A 12 ml d'une solution 1 molaire de chlorure de thionyle dans le dichlorométhane, refroidie à la température de 0°C, on ajoute successivement 6 ml d'une solution 2 molaire de pyrazine dans le dichlorométhane et 5 ml d'une solution 2 molaire (10 mmoles) de benzaldéhyde dans le dichlorométhane. On agite le mélange à 0°C pendant six heures et on procède ensuite comme en a) ci-dessus.
   Rendement: 50%.

On prépare selon la méthode de l'exemple 1.a) les composés réunis dans le tableau I.

**TABLEAU I**

| 2-R₁ -5-R₂ -1H-benzimidazoles. | | | | |
|---|---|---|---|---|
| Exemple | R₁ | R₂ | P.F. (°C) | Rendement (%) |
| 2 | 4-CH₃-C₆H₄- | H | 210-212 | 80 |
| 3 | 4-NO₂-C₆H₄- | H | 298-299 | 55 |
| 4 | 2-thiényle | H | 287-290 | 75 |
| 5 | 3-pyridinyle | H | 254-255 | 80 |
| 6 | 4-Cl-C₆H₄- | Cl | 217-220 | 65 |
| 7 | tert-butyle | H | >300 | 70 |

On prépare également selon la méthode de l'exemple 1.b) les composés réunis dans le tableau II.

**TABLEAU II**

| 2-R₁ -5-R₂ -1H-benzimidazoles. | | | | |
|---|---|---|---|---|
| Exemple | R₁ | R₂ | P.F. (°C) | Rendement (%) |
| 8 | 4-CN-C₆H₄- | Cl | 265-267 | 70 |
| 9 | 4-NO₂-C₆H₄- | Cl | 263-265 | 80 |
| 10 | 4-Cl-C₆H₄- | NO₂ | > 300 | 70 |
| 11 | 4-F-C₆H₄- | Cl | 223-225 | 65 |

### Exemple 12. Préparation du 5-chloro-2-(4-chlorophényl)-1H-benzimidazole.

Ce composé qui a déjà été préparé à l'exemple 6 peut aussi être obtenu de la manière suivante.

Dans 50 ml de toluène, on ajoute successivement 8 ml (110 mmoles) de chlorure de thionyle, 8,88 ml (110 mmoles) de pyridine et 14,06 g (100 mmoles) de 4-chlorobenzaldéhyde. La réaction est exothermique et le mélange de réaction se sépare en deux phases liquides. On introduit ensuite 14,3 g (100 mmoles) de 4-chloro-1,2-benzènediamine. On ajoute enfin 50 ml d'une solution aqueuse contenant 16,4 g (200 mmoles) d'acétate de sodium et on chauffe le mélange réactionnel à reflux pendant une heure. Le produit de la réaction précipite. On le filtre et on le lave avec un mélange eau-alcool (1/1). On obtient le 5-chloro-2-(4-chlorophényl)-1H-benzimidazole avec un rendement de 75%.

### Exemple 13. Préparation du 2-(2-chlorophényl)-1H-benzimidazole.

Ce composé est préparé selon la méthode de l'exemple 1.a) à partir de 2-chlorobenzaldéhyde et de 1,2-benzènediamine, mois on n'opère pas sous atmosphère inerte. Lorsque la réaction est terminée, on filtre le chlorhydrate de 1,2-benzènediamine qui s'est formé. Ensuite, on évapore le filtrat sous pression réduite. On traite le résidu par de l'eau, et le 2-(2-chlorophényl)-1H-benzimidazole précipite. P.F. 225-227°C. Rendement : 55%.

### Exemple 14. Préparation du 2-(2-furanyl)-1H-benzimidazole.

Dans 20 ml de pyridine refroidis à 0°C, et sous atmosphère d'azote, on ajoute successivement 0,88 ml (12 mmoles) de chlorure de thionyle et 0,83 ml (10 mmoles) de 2-furanecarboxaldéhyde. On continue ensuite comme à l'exemple 1.a). On obtient le 2-(2-furanyl)-1H-benzimidazole de P.F. 285-287°C. Rendement : 95%.

### Exemple 15. Préparation du 5-nitro-2-(4-nitrophényl)-1H-benzimidazole.

A une solution contenant 16 ml (220 mmoles) de chlorure de thionyle dans 500 ml de dichlorométhane, on ajoute successivement à la température ambiante 19 ml (220 mmoles) de pyridine et 30,2 g (220 mmoles) de 4-nitrobenzaldéhyde. On agite le mélange pendant une heure. On ajoute ensuite, lentement, 61,2 g (400 mmoles) de 4-nitro-1,2-benzènediamine et on maintient encore l'agitation pendant plusieurs heures. On filtre le précipité qui s'est formé et on le lave abondamment à l'eau bouillante afin d'éliminer le chlorhydrate de 4-nitro-1,2-benzènediamine qui est peu soluble. On obtient ainsi le 5-nitro-2-(4-nitrophényl)-1H-benzimidazole, qui fond à plus de 300°C. Rendement : 70%.

A partir de ce composé on peut préparer la 2-(4-aminophényl)-1H-benzimidazol-5-amine (matière première utilisable dans la fabrication de polyimides) par réduction en opérant comme suit :

On ajoute par petites portions 13,5 g (60 mmoles) de chlorure stanneux à une solution de 2,8 g (10 mmoles) de 5-nitro-2-(4-nitrophényl)-1H-benzimidazole dans 15 ml d'acide chlorhydrique concentré. La réaction est très exothermique. Après l'addition, on chauffe le mélange réactionnel à reflux pendant quatre heures. Ensuite on refroidit à la température ambiante et on précipite le chlorhydrate de 2-(4-aminophényl)-1H-benzimidazol-5-amine par addition d'alcool éthylique au milieu réactionnel. On filtre le chlorhydrate et on le redissout dans de l'eau. La 2-(4-aminophényl)-1H-benzimidazol-5-amine est ensuite précipitée par addition d'ammoniaque. Après filtration et séchage, on obtient un produit de P.F. 151-153°C. Rendement : 75%.

### Exemple 16. Préparation du 2-n-propyl-1H-benzimidazole.

Ce composé est préparé selon la méthode de l'exemple 1.a), mais à partir de butyraldéhyde et de 1,2-benzènediamine. Lorsque la réaction est terminée, on filtre le chlorhydrate de 1,2-benzènediamine qui s'est formé et on le lave avec un peu de dichlorométhane. Ensuite, on évapore le filtrat sous pression réduite. On traite le résidu par de l'eau et le 2-n-propyl-1H-benzimidazole cristallise. P.F. 160-163°C.
Rendement : 50%.

On prépare de la même manière les composés rassemblés dans le tableau III.

**TABLEAU III**

| 2-R₁ -5-R₂ -1H-benzimidazoles. | | | | |
|---|---|---|---|---|
| Exemple | R₁ | R₂ | P.F. (°C) | Rendement (%) |
| 17 | 1-méthyléthyle | H | 224-225 | 45 |
| 18 | benzyle | H | 185-187 | 50 |
| 19 | n-nonyle | H | 125-128 | 60 |

### Exemple 20. Préparation du 2-benzyl-1H-benzimidazole.

Ce composé qui a déjà été préparé à l'exemple 18 peut aussi être obtenu en effectuant la réaction dans le toluène de la manière suivante.

A la température de 0°C, et sous atmosphère inerte (azote), on mélange successivement 12 ml d'une solution 1 molaire de chlorure de thionyle dans le toluène, 6 ml d'une solution 2 molaire de pyridine dans le toluène et finalement 5 ml d'une solution 2 molaire (10 mmoles) de phénylacétaldéhyde dans le même solvant. On termine ensuite comme à l'exemple 15. On obtient ainsi le 2-benzyl-1H-benzimidazole de P.F. 185-187°C. Rendement : 60%.

### Exemple 21. Préparation de l'acide 2-(4-carboxyphényl)-1H-benzimidazole-5-carboxylique.

A 1,7 ml (22 mmoles) de chlorure de thionyle dissous dans 25 ml de dichlorométhane, on ajoute successivement, à la température ambiante, 1,9 ml (22 mmoles) de pyridine et 1,5 g (10 mmoles) d'acide 4-formylbenzoïque. On agite le mélange pendant une heure, puis on ajoute lentement 4,6 g (30 mmoles) d'acide 3,4-diaminobenzoïque et on maintient encore l'agitation pendant plusieurs heures. On filtre le précipité qui s'est formé au cours de la réaction, on le lave abondamment avec de l'eau, puis on le redissout dans une solution aqueuse d'hydroxyde de sodium. L'acide 2-(4-carboxyphényl)-1H-benzimidazole-5-carboxylique précipite par acidification de la solution aqueuse avec une solution d'acide chlorhydrique diluée. Le produit obtenu fond à plus de 300°C.
Rendement : 90%.

### Exemple 22. Préparation du 2,2'-(1,3-phénylène)bis(1H-benzimidazole).

Ce composé est préparé selon la méthode de l'exemple 1.a), mais à partir de 0,77 g (5 mmoles) de 1,3-benzènedicarboxaldéhyde et de 3,24 g (30 mmoles) de 1,2-benzènediamine. P.F.: 225-226°C. Rendement: 50%.

### Exemple 23. Préparation du 2,2′-(1,4-phénylène)bis(5-chloro-1H-benzimidazole).

Ce composé est préparé selon la méthode de l'exemple 1.b) mais au départ de 1,34 g (10 mmoles) de 1,4-benzènedicarboxaldéhyde et de 8,6 g (60 mmoles) de 4-chloro-1,2-benzènediamine. P.F. : plus de 300°C.
Rendement : 85%.

### Exemple 24. Préparation du 2,2′-bis(4-chlorophényl)-5,5′-bi-1H-benzimidazole.

Dans 50 ml de dichlorométhane on ajoute successivement 1,6 ml (22 mmoles) de chlorure de thionyle, 1,8 ml (22 mmoles) de pyridine et 2,8 g (20 mmoles) de 4-chlorobenzaldéhyde. On agite le mélange pendant une heure, ensuite on ajoute lentement 6,4 g (60 mmoles) de [1,1′-biphényl]-3,3′,4,4′-tétramine et on poursuit l'agitation pendant plusieurs heures. On filtre le précipité qui s'est formé pendant la réaction, on le lave abondamment avec de l'eau et on le dissout ensuite dans du N,N-diméthylformamide. On filtre le chlorhydrate de [1,1′-biphényl]-3,3′,4,4′-tétramine qui est insoluble et on précipite le 2,2′-bis(4-chlorophényl)-5,5′-bi-1H-benzimidazole en ajoutant de l'eau au filtrat. P.F. : plus de 300°C. Rendement : 55%.

### Exemple 25. Préparation de l'acide 2,2′-(1,4-phénylène)bis(1H-benzimidazole-5-carboxylique).

Dans 50 ml de toluène, on ajoute successivement 1,6 ml (22 mmoles) de chlorure de thionyle, 1,8 ml (22 mmoles) de pyridine et 1,34 g (10 mmoles) de 1,4-benzènedicarboxaldéhyde. On agite le mélange pendant une heure, ensuite on ajoute lentement 9,15 g (60 mmoles) d'acide 3,4-diaminobenzoïque et on chauffe le mélange à l'ébullition pendant 6 heures. Le précipité d'acide 2,2′-(1,4-phénylène)bis(1H-benzimidazole-5-carboxylique) qui s'est formé est séparé par filtration. On lave le précipité à l'eau et finalement avec de l'alcool bouillant. P.F. : plus de 300°C. Rendement : 60%.

### Exemple 26. Préparation de l'acide 2,2′-(1,3-phénylène)bis(1H-benzimidazole-5-carboxylique).

Ce composé est préparé selon la méthode de l'exemple 25 mais en remplaçant le 1,4-benzènedicarboxaldéhyde par le 1,3-benzènedicarboxaldéhyde.

L'acide 2,2′-(1,3-phénylène)bis(1H-benzimidazole-5-carboxylique) fond à plus de 300°C. Rendement : 80%.

### Exemple 27. Préparation de l'acide 4,4′-[5,5′-bi-1H-benzimidazole]-2,2′-diylbis(benzoïque).

Ce composé est préparé selon la méthode de l'exemple 21, mais après avoir agité le mélange réactionnel pendant une heure, on ajoute lentement une solution contenant 2,05 g (25 mmoles) d'acétate,de sodium dans 25 ml d'eau et ensuite 1,07 g (5 mmoles) de [1,1′-biphényl]-3,3′,4,4′-tétramine.

L'acide 4,4′-[5,5′-bi-1H-benzimidazole]-2,2′-diylbis(benzoïque) obtenu fond à plus de 300°C. Rendement : 85%.

## Revendications

1. Procédé de préparation de 1H-benzimidazoles substitués en position 2, à partir d'une 1,2-benzènediamine et d'un aldéhyde, caractérisé en ce que l'on fait d'abord réagir ensemble un aldéhyde avec un halogénure de thionyle et un composé hétérocyclique aromatique azoté choisi dans le groupe consistant en la pyridine, la pyrimidine, la quinoléine, l'isoquinoléine, la pyrazine et le 1-méthylimidazole, en ce que l'on fait réagir ensuite, en présence d'un agent fixateur d'acide le produit ainsi formé avec une 1,2-benzènediamine éventuellement substituée et en ce qu'on sépare le 1H-benzimidazole ainsi obtenu du mélange de réaction.

2. Procédé selon la revendication 1, pour la préparation de 1H-benzimidazoles substitués en position 2 répondant à la formule générale dans laquelle 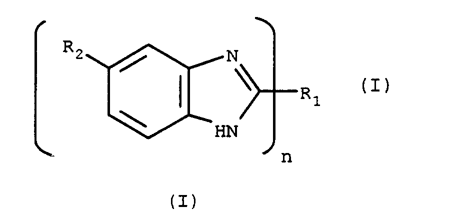
R₁ représente un radical alkyle linéaire ou ramifié contenant 1 à 13 atomes de carbone, un radical phényle, halogénophényle, 4-nitrophényle, 4-méthylphényle, 4-cyanophényle, 4-carboxyphényle, benzyle, thiényle, furanyle, pyridinyle ou un radical 1,3- ou 1,4-phénylène,
R₂ représente un atome d'hydrogène ou de chlore, un groupe nitro ou carboxyle, le groupe 2-(4-chlorophényl)-1H-benzimidazol-5-yle, ou le groupe 2-(4-carboxyphényl)-1H-benzimidazole-5-yle, et n est égal à 1 ou 2,
avec les restrictions que :
a) lorsque R₁ représente un radical alkyle, phényle, 4-méthylphényle, benzyle, thiényle, furanyle ou pyridinyle, R₂ est de l'hydrogène et n = 1,
b) lorsque R₁ représente un radical halogénophényle ou 4-nitrophényle, R₂ est un atome d'hydrogène ou de chlore ou un groupe nitro et n = 1,
c) lorsque R₁ représente le radical 4-cyanophényle, R₂ représente un atome de chlore et n = 1,
d) lorsque R₁ représente le radical 4-carboxyphényle, R₂ représente un groupe carboxyle ou le groupe 2-(4-carboxyphényl)-1H-benzimidazol-5-yle et n = 1,
e) lorsque R₁ est un radical 1,3- ou 1,4-phénylène, R₂ représente un atome d'hydrogène ou de chlore ou un groupe carboxyle et n = 2, et
f) lorsque R₂ est le groupe 2-(4-chlorophényl)-1H-benzimidazol-5-yle, R₁ représente le radical 4-chlorophényle et n = 1,
caractérisé en ce que l'on fait réagir ensemble un aldéhyde de formule
R₁(CHO)ₙ (II)
dans laquelle R₁ et n ont la signification donnée ci-dessus, avec un halogénure de thionyle et un composé hétérocyclique aromatique azoté choisi dans le groupe consistant en la pyridine, la pyrimidine, la quinoléine, l'isoquinoléine, la pyrazine et le 1-méthylimidazole, en ce que l'on fait réagir ensuite, en présence d'un agent fixateur d'acide le produit ainsi formé (a) avec une 1,2- benzènediamine de formule 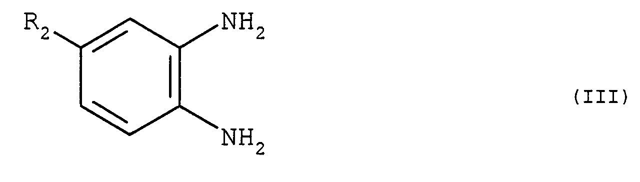 dans laquelle R₂ représente un atome d'hydrogène ou de chlore ou un groupe nitro ou carboxyle lorsqu'on désire obtenir un 1H-benzimidazole de formule I dans laquelle R₂ représente un atome d'hydrogène ou de chlore ou un groupe nitro ou carboxyle, ou (b) avec la [1,1'-biphényl]-3,3',4,4'-tétramine lorsqu'on désire obtenir un 1H-benzimidazole de formule I dans laquelle R₂ représente le groupe 2-(4-chlorophényl)-1H-benzimidazol-5-yle ou le groupe 2-(4-carboxyphényl)-1H-benzimidazol-5-yle et en ce qu'on sépare le 1H-benzimidazole ainsi obtenu du mélange de réaction.

3. Procédé selon l'une quelconque des revendications 1 et 2, caractérisé en ce que la réaction est exécutée dans un solvant inerte choisi parmi les hydrocarbures aliphatiques, cycloaliphatiques ou aromatiques, les hydrocarbures aliphatiques, cycloaliphatiques ou aromatiques chlorés et les éthers aliphatiques.

4. Procédé selon l'une quelconque des revendications 1 à 3, caractérisé en ce que la réaction est exécutée à une température comprise entre 0°C et la température d'ébullition du solvant.

5. Procédé selon l'une quelconque des revendications 1 à 4, caractérisé en ce que en tant qu'agent fixateur d'acide on utilise la 1,2-benzènediamine en excès.

6. Procédé selon l'une quelconque des revendications 1 à 4, caractérisé en ce que en tant qu'agent fixateur d'acide on utilise l'acétate de sodium.

7. Procédé selon l'une quelconque des revendications 1 à 6, caractérisé en ce que l'halogénure de thionyle est le chlorure ou le bromure de thionyle.

8. Procédé selon l'une quelconque des revendications 1 à 7, caractérisé en ce que le composé hétérocyclique aromatique azoté est la pyridine.

9. Procédé selon l'une quelconque des revendications 1 à 8, caractérisé en ce que l'aldéhyde est choisi parmi les aldéhydes aliphatiques linéaires ou ramifiés contenant 2 à 14 atomes de carbone, le benzaldéhyde, le 2-chlorobenzaldéhyde, le 4-chlorobenzaldéhyde, le 4-nitrobenzaldéhyde, le 4-méthylbenzaldéhyde, le 4-cyanobenzaldéhyde, le phénylacétaldéhyde, l'acide 4-formylbenzoïque, le 2- ou 3-pyridinecarboxaldéhyde, le 2- ou 3-furanecarboxaldéhyde, le 2- ou 3-thiophènecarboxaldéhyde, et le 1,3 ou 1,4-benzènedicarboxaldéhyde.

10. Procédé selon l'une quelconque des revendications 1 à 9, caractérisé en ce que la 1,2-benzènediamine est choisie parmi la 1,2-benzènediamine, la 4-chloro-1,2-benzènediamine, la 4-nitro-1,2-benzènediamine, l'acide 3,4-diaminobenzoïque et la [1,1'-biphényl]-3,3',4,4'-tétramine.

## Claims

1. Process for the preparation of 1H-benzimidazole substituted in position 2, from a 1,2-benzenediamine and an aldehyde, characterised in that an aldehyde is firstly reacted with a thionyl halide and a nitrogenous aromatic heterocyclic compound, selected from the group consisting of pyridine, pyrimidine, quinoline, isoquinoline, pyrazine and 1-methylimidazole, in that the product formed is then reacted in the presence of an acid fixing agent with a 1,2-benzenediamine, optionally substituted, and in that the resulting 1H-benzimidazole is separated from the reaction mixture.

2. Process according to claim 1, for the preparation of 1H-benzimidazole substituted in position 2 and having the general formula: 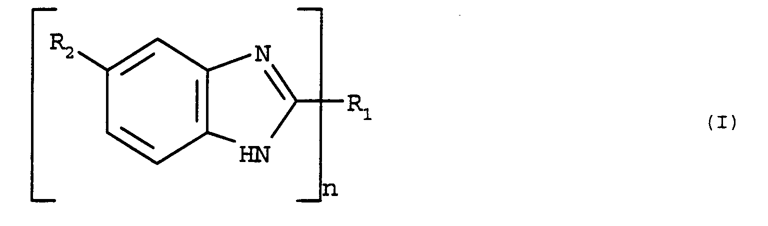 wherein
R₁ represents a straight-chain or branched alkyl radical containing 1 to 13 carbon atoms, a phenyl, halophenyl, 4-nitrophenyl, 4-methylphenyl, 4-cyanophenyl, 4-carboxyphenyl, benzyl, thienyl, furanyl or pyridinyl radical, or a 1,3- or 1,4-phenylene radical,
R₂ represents a hydrogen or chlorine atom, a nitro or carboxyl group, a 2-(4-chlorophenyl)-1H-benzimidazol-5-yl group or a 2-(4-carboxyphenyl)-1H-benzimidazol-5-yl group and n is equal to 1 or 2,
with the proviso that:
a) when R₁ represents an alkyl, phenyl, 4-methylphenyl, benzyl, thienyl, furanyl or pyridinyl radical, R₂ is hydrogen and n=1,
b) when R₁ represents a halophenyl or 4-nitrophenyl radical, R₂ is hydrogen or chlorine atom or a nitro group and n=1,
c) when R₁ represents a 4-cyanophenyl radical, R₂ represents a chlorine atom and n=1,
d) when R₁ represents a 4-carboxyphenyl radical, R₂ represents a carboxyl group or a 2-(4-carboxyphenyl)-1H-benzimidazol-5-yl group and n=1,
e) when R₁ is a 1,3- or 1,4-phenyl radical, R₂ represents a hydrogen or chlorine atom or a carboxyl group and n=2, and
f) when R₂ is a 2-(4-chlorophenyl)-1H-benzimidazol-5-yl group, R₁ represents a 4-chlorophenyl radical and n=1,
characterised in that an aldehyde having the formula
R₁(CHO)ₙ (II)
in which R₁ and n have the meaning given above, is reacted with a thionyl halide and a nitrogenous aromatic heterocyclic compound selected from the group consisting of pyridine, pyrimidine, quinoline, isoquinoline, pyrazine and 1-methylimidazole and the so obtained product is then reacted in the presence of an acid fixing agent with
(a) a 1,2-benzenediamine having the formula: 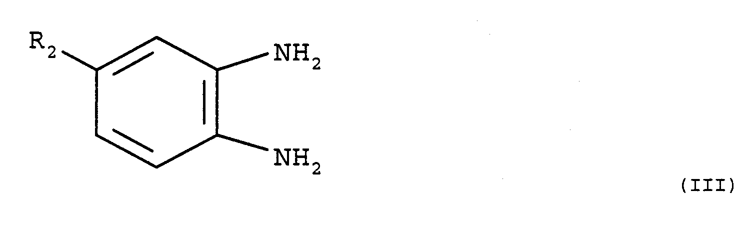 in which R₂ represents a hydrogen or chlorine atom or a nitro or carboxyl group when it is desired to obtain a 1H-benzimidazole of formula I wherein R₂ represents a hydrogen or chlorine atom or a nitro or carboxyl group, or
(b) with [1,1'-biphenyl]-3,3',4,4'-tetramine, when it is desired to obtain a 1H-benzimidazole of formula I wherein R₂ represents a 2-(4-chlorophenyl)-1H-benzimidazol-5-yl group or a 2-(4-carboxyphenyl)-1H-benzimidazol-5-yl group and in that the resulting 1H-benzimidazole is separated from the reaction mixture.

3. Process according to any of claims 1 and 2, characterised in that the reaction is brought about in an inert solvent selected from aliphatic, cycloaliphatic or aromatic hydrocarbons, chlorinated aliphatic, cycloaliphatic or aromatic hydrocarbons and aliphatic ethers.

4. Process according to any of claims 1 to 3, characterised in that the reaction is brought about at a temperature between 0°C and the boiling point of the solvent.

5. Process according to any of claims 1 to 4, characterised in that an excess of 1,2-benzenediamine is used as an acid fixing agent.

6. Process according to any of claims 1 to 4, characterised in that sodium acetate is used as an acid fixing agent.

7. Process according to any of claims 1 to 6, characterised in that the thionyl halide is thionyl chloride or bromide.

8. Process according to any of claims 1 to 7, characterised in that the nitrogenous aromatic heterocyclic compound is pyridine.

9. Process according to any of claims 1 to 8, characterised in that the aldehyde is selected from straight-chain or branched aliphatic aldehydes containing 2 to 14 carbon atoms, benzaldehyde, 2-chlorobenzaldehyde, 4-chlorobenzaldehyde, 4-nitrobenzaldehyde, 4-methylbenzaldehyde, 4-cyanobenzaldehyde, phenylacetaldehyde, 4-formylbenzoic acid, 2- or 3-pyridinecarboxaldehyde, 2- or 3-furancarboxyaldehyde, 2- or 3-thiophenecarboxaldehyde, or 1,3- or 1,4-benzenedicarboxaldehyde.

10. Process according to any of claims 1 to 9, characterised in that the 1,2-benzenediamine is selected from 1,2-benzenediamine, 4-chloro-1,2-benzenediamine, 4-nitro-1,2-benzenediamine, 3,4-diaminobenzoic acid or [1,1'-biphenyl]-3,3',4,4'-tetramine.

## Patentansprüche

1. Verfahren zur Herstellung von in 2-Position substituierten 1H-Benzimidazolen aus einem 1,2-Diaminobenzol und einem Aldehyd, dadurch gekennzeichnet, daß man zuerst einen Aldehyd zusammen mit einem Thionylhalogenid und einer stickstoffhaltigen aromatischen heterozyklischen Verbindung, die aus der aus Pyridin, Pyrimidin, Chinolin, Isochinolin, Pyrazin und 1-Methylimidazol bestehenden Gruppe ausgewählt ist, umsetzt, dadurch, daß man dann das so gebildete Produkt in Gegenwart eines säurebindenden Mittels mit einem gegebenenfalls substituierten 1,2-Diaminobenzol umsetzt, und dadurch, daß man das so erhaltene 1H-Benzimidazol von dem Reaktionsgemisch abtrennt.

2. Verfahren gemäß Anspruch 1 zur Herstellung von in 2-Position substituierten 1H-Benzimidazolen, die der allgemeinen Formel 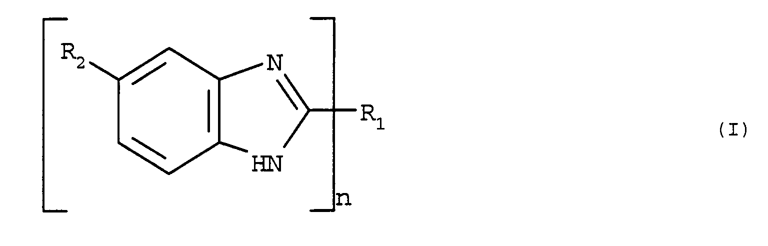 entsprechen, in welcher
R₁ einen linearen oder verzweigten Alkylrest mit 1 bis 13 Kohlenstoffatomen, einen Phenyl-, Halogenphenyl-, 4-Nitrophenyl-, 4-Methylphenyl-, 4-Cyanphenyl-, 4-Carboxyphenyl-, Benzyl-, Thienyl-, Furyl-, Pyridyl- oder einen 1,3- oder 1,4-Phenylenrest darstellt,
R₂ ein Wasserstoff- oder Chloratom, eine Nitro- oder Carboxylgruppe, die 2-(4-Chlorphenyl)-1H-benzimidazol-5-yl-Gruppe oder die 2-(4-Carboxyphenyl)-1H-benzimidazol-5-yl-Gruppe darstellt und n gleich 1 oder 2 ist,
mit den Beschränkungen, daß
a) wenn R₁ einen Alkyl-, Phenyl-, 4-Methylphenyl-, Benzyl-, Thienyl-, Furyl- oder Pyridylrest darstellt, R₂ Wasserstoff ist und n gleich 1 ist,
b) wenn R₁ einen Halogenphenyl- oder 4-Nitrophenylrest darstellt, R₂ ein Wasserstoff- oder Chloratom oder eine Nitrogruppe ist und n gleich 1 ist,
c) wenn R₁ den 4-Cyanphenylrest darstellt, R₂ ein Chloratom darstellt und n gleich 1 ist,
d) wenn R₁ den 4-Carboxyphenylrest darstellt, R₂ eine Carboxylgruppe oder die 2-(4-Carboxyphenyl)-1H-benzimidazol-5-yl-Gruppe darstellt und n gleich 1 ist,
e) wenn R₁ einen 1,3- oder 1,4-Phenylenrest datstellt, R₂ ein Wasserstoff- oder Chloratom oder eine Carboxylgruppe darstellt und n gleich 2 ist, und
f) wenn R₂ die 2-(4-Chlorphenyl)-1H-benzimidazol-5-yl-Gruppe ist, R₁ den 4-Chlorphenylrest darstellt und n gleich 1 ist,
dadurch gekennzeichnet, daß man einen Aldehyd der Formel
R₁(CHO)ₙ (II)
in welcher R₁ und n die oben angegebene Bedeutung haben, zusammen mit einem Thionylhalogenid und einer stickstoffhaltigen aromatischen heterozyklischen Verbindung, die aus der aus Pyridin, Pyrimidin, Chinolin, Isochinolin, Pyrazin, 1-Methylimidazol bestehenden Gruppe ausgewählt ist, umsetzt, dadurch, daß man dann das so gebildete Produkt in Gegenwart eines säurebindenden Mittels (a) mit einem 1,2-Diaminobenzol der Formel 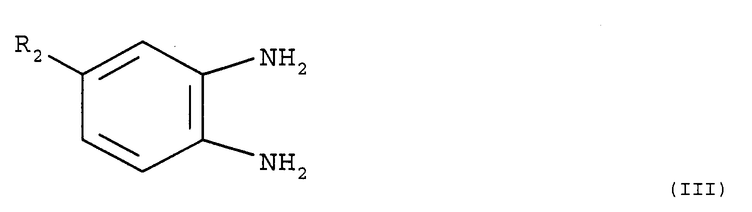 in welcher R₂ ein Wasserstoff- oder Chloratom oder eine Nitro- oder Carboxylgruppe darstellt,
umsetzt, wenn man ein 1H-Benzimidazol der Formel (I) erhalten möchte, in welcher R₂ ein Wasserstoff- oder Chloratom oder eine Nitro- oder Carboxylgruppe darstellt, oder (b) mit [1,1'-Biphenyl]-3,3',4,4'-tetramin umsetzt, wenn man ein 1H-Benzimidazol der Formel (I) erhalten möchte, in welcher R₂ die 2-(4-Chlorphenyl)-1H-benzimidazol-5-yl-Gruppe oder die 2-(4-Carboxyphenyl)-1H-benzimidazol-5-yl-Gruppe darstellt, und dadurch, daß man das so erhaltene 1H-Benzimidazol vom Reaktionsgemisch abtrennt.

3. Verfahren gemäß einem der Ansprüche 1 und 2, dadurch gekennzeichnet, daß die Reaktion in einem inerten Lösungsmittel ausgeführt wird, das unter den aliphatischen, cycloaliphatischen oder aromatischen Kohlenwasserstoffen, den chlorierten aliphatischen, cycloaliphatischen oder aromatischen Kohlenwasserstoffen und den aliphatischen Ethern ausgewählt ist.

4. Verfahren gemäß einem der Ansprüche 1 bis 3, dadurch gekennzeichnet, daß die Reaktion bei einer Temperatur zwischen 0°C und der Siedetemperatur des Lösungsmittels ausgeführt wird.

5. Verfahren gemäß einem der Ansprüche 1 bis 4, dadurch gekennzeichnet, daß man als säurebindendes Mittel 1,2-Diaminobenzol im Überschuß verwendet.

6. Verfahren gemäß einem der Ansprüche 1 bis 4, dadurch gekennzeichnet, daß man als säurebindendes Mittel Natriumacetat verwendet.

7. Verfahren gemäß einem der Ansprüche 1 bis 6, dadurch gekennzeichnet, daß Thionylhalogenid Thionylchlorid oder -bromid ist.

8. Verfahren gemäß einem der Ansprüche 1 bis 7, dadurch gekennzeichnet, daß die stickstoffhaltige aromatische heterozyklische Verbindung Pyridin ist.

9. Verfahren gemäß einem der Ansprüche 1 bis 8, dadurch gekennzeichnet, daß der Aldehyd unter den linearen oder verzweigten aliphatischen Aldehyden mit 2 bis 14 Kohlenstoffatomen, Benzaldehyd, 2-Chlorbenzaldehyd, 4-Chlorbenzaldehyd, 4-Nitrobenzaldehyd, 4-Methylbenzaldehyd, 4-Cyanbenzaldehyd, Phenylacetaldehyd, 4-Carboxybenzaldehyd, Pyridin-2- oder Pyridin-3-carbaldehyd, Furan-2- oder Furan-3-carbaldehyd, Thiophen-2- or Thiophen-3-carbaldehyd, und Benzol-1,3- oder Benzol-1,4-dicarbaldehyd ausgewählt ist.

10. Verfahren gemäß einem der Ansprüche 1 bis 9, dadurch gekennzeichnet, daß das 1,2-Diaminobenzol unter 1,2-Diaminobenzol, 4-Chlor-1,2-diaminobenzol, 1,2-Diamino-4-nitrobenzol, 3,4-Diaminobenzoesäure und [1,1'-Biphenyl]-3,3',4,4'-tetramin ausgewählt ist.
